# EUROPEAN PATENT APPLICATION

(11) **EP 1 589 117 A2**
(43) Date of publication of application: **26.10.2005**
(21) Application number: 05252152.3
(22) Date of filing: 06.04.2005
(51) Int. Cl.: C12Q 1/68

(54) **Methods for determining the relationship between hybridization signal of probes and target DNA copy number**

(30) Priority: 20.04.2004 US 828907
(71) Applicant: Agilent Technologies, Inc., Palo Alto, CA 94306 (US)
(72) Inventor: Barrett, Michael T., Montain View, CA 94040 (US); Scheffer, Alicia F., Redwood City, CA 94061 (US)
(74) Representative: Tollett, Ian

(57) **Abstract**

Methods for evaluating surface-bound polynucleotides are provided. Specifically, the methods involve contacting an array of surface-bound polynucleotides with a population of labeled nucleic acids made from a non-naturally occurring composition of chromosomes, and evaluating binding of the labeled nucleic acids to a surface-bound polynucleotide. The methods may be used, for example, to screen for surface bound polynucleotides that have desirable binding characteristics, e.g., suitability for use in array-based comparative genomic hybridization assays. Kits and computer programming for use in practising the subject methods are also provided.

## Description

Many genomic and genetic studies are directed to the identification of differences in gene dosage or expression among cell populations for the study and detection of disease. For example, many malignancies involve the gain or loss of DNA sequences resulting in activation of oncogenes or inactivation of tumor suppressor genes. Identification of the genetic events leading to neoplastic transformation and subsequent progression can facilitate efforts to define the biological basis for disease, improve prognosis of therapeutic response, and permit earlier tumor detection. In addition, perinatal genetic problems frequently result from loss or gain of chromosome segments such as trisomy 21 or the micro deletion syndromes. Thus, methods of prenatal detection of such abnormalities can be helpful in early diagnosis of disease.

Comparative genomic hybridization (CGH) is one approach that has been employed to detect the presence and identify the location of amplified or deleted sequences. In one implementation of CGH, genomic DNA is isolated from normal reference cells, as well as from test cells (e.g., tumor cells). The two nucleic acids are differentially labeled and then simultaneously hybridized *in situ* to metaphase chromosomes of a reference cell. Chromosomal regions in the test cells which are at increased or decreased copy number can be identified by detecting regions where the ratio of signal from the two distinguishably labeled nucleic acids is altered. For example, those regions that have been decreased in copy number in the test cells will show relatively lower signal from the test nucleic acid than the reference compared to other regions of the genome. Regions that have been increased in copy number in the test cells will show relatively higher signal from the test nucleic acid.

In a recent variation of the above traditional CGH approach, the immobilized chromosome element has been replaced with a collection of solid support surface-bound polynucleotides, e.g., an array of BAC (bacterial artificial chromosome) clones or cDNAs. Such approaches offer benefits over immobilized chromosome approaches, including a higher resolution, as defined by the ability of the assay to localize chromosomal alterations to specific areas of the genome.

Despite great interest in CGH technology, methods for empirically evaluating and identifying suitable surface-bound polynucleotides for use in this technology are limited. A rigorous method would be to measure signals (e.g. ratios) from each polynucleotide in controlled experiments with test samples containing known copy numbers for each sequence on the array. For example, a widely used method for assaying polynucleotides that are specific for sequences on the X chromosome is to use a series of cell lines with known variable copies of that chromosome for CGH experiments. These cell lines (X series) contain intact copies (e.g. 1 to 5) of the X chromosome permitting a rigorous measure of the relationship between copy number and signal intensities for each X chromosome specific polynucleotide on an array. However, cell lines containing known variable numbers of intact copies of each of the other chromosomes in the genome are generally not available. Furthermore, the X series cell lines are slow growing and can spontaneously vary in ploidy under standard culturing conditions. Thus, such methods cannot readily be used to assay the relationship between the hybridization signal of polynucleotides and the genomic copy number of sequences from each chromosome in a cell .

Accordingly, a great need exists for methods for evaluating surface-bound CGH probe nucleic acids. This invention meets this, and other, needs.

### Relevant Literature

United States Patents of interest include: 6,465,182; 6,335,167; 6,251,601; 6,210,878; 6,197,501; 6,159,685; 5,965,362; 5,830,645; 5,665,549; 5,447,841 and 5,348,855. Also of interest are published United States Application Serial No. 2002/0006622 and published PCT application WO 99/23256. Articles of interest include: Pollack et al., Proc. Natl. Acad. Sci. (2002) 99: 12963-12968; Wilhelm et al., Cancer Res. (2002) 62: 957-960; Pinkel et al., Nat. Genet. (1998) 20: 207-211; Cai et al., Nat. Biotech. (2002) 20: 393-396; Snijders et al., Nat. Genet. (2001) 29:263-264; Hodgson et al., Nat. Genet. (2001) 29:459-464; and Trask, Nat. Rev. Genet. (2002) 3: 769-778

Methods for evaluating surface-bound polynucleotides are provided. Specifically, the methods involve contacting an array of surface-bound polynucleotides with a population of labeled nucleic acids made from a non-naturally occurring composition of chromosomes, and evaluating binding of the labeled nucleic acids to a surface-bound polynucleotide. In most embodiments, binding is evaluated relative to binding of a second population of labeled nucleic acids made from a reference composition of chromosomes. The methods may be used to screen for surface bound polynucleotides that have desirable binding characteristics, e.g., suitability for use in array-based comparative genomic hybridization assays. Kits and computer programming for use in practicing the subject methods are also provided.
Fig. 1 is a schematic representation of an embodiment of the subject methods.
Fig. 2 is a schematic representation of another embodiment of the subject methods.

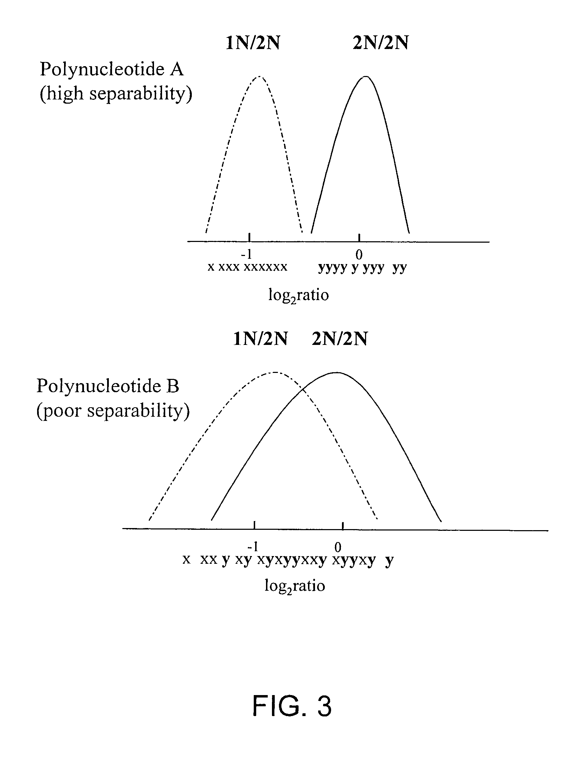

Fig 3 is two panels of graphs showing the separability of the distributions of the signals (e.g. ratios) from individual probes with desirable binding characteristics (polynucleotide A); and non-desirable binding characteristics (polynucleotide B). Identification of surface bound polynucleotides with desirable binding characteristics are identified by the separability of the distributions of their signals (e.g. ratios) in comparisons of two or more chromosome composition ratios (1N/2N, 2N/2N) in multiple repeat (n=10) hybridizations. Polynucleotide A with high separability has desirable binding characteristics. x: observed ratio for IN/2N in single experiment. y: Observed ratio for 2N/2N in single experiment.
Fig. 4 is a graph showing data from a hybridization with a non-cellular composition containing the equivalent of 4 copies of chromosome 17 in the test channel, and a sample with the equivalent of 2 copies of each chromosome, including 17, in the reference channel. Test sample is a non-cellular composition containing the equivalent of 4 copies of chromosome 17. Reference sample has normal 2 copy content. Ideal log ratio = 0.3 . This array contains probes for chromosomes 16, 17, 18 and X

The term "nucleic acid" and "polynucleotide" are used interchangeably herein to describe a polymer of any length composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length. Oligonucleotides are usually synthetic and, in many embodiments, are under 50 nucleotides in length.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other nucleic acids that are C-glycosides of a purine or pyrimidine base, polypeptides (proteins), polysaccharides (starches, or polysugars), and other chemical entities that contain repeating units of like chemical structure.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "surface-bound polynucleotide" refers to a polynucleotide that is immobilized on a surface of a solid substrate, where the substrate can have a variety of configurations, e.g., a sheet, bead, or other structure. In certain embodiments, the collections of oligonucleotide target elements employed herein are present on a surface of the same planar support, e.g., in the form of an array.

A "surface-bound polynucleotide with desirable binding characteristics", as discussed in greater detail below, refers to a surface-bound polynucleotide that has properties that make it suitable for array-based comparative genome hybridization experiments. Such polynucleotides usually exhibit an observed binding behavior that is similar to an expected binding behavior. For example, if binding of a surface-bound polynucleotide to its target sequence is expected to be linear then that polynucleotide is a surface-bound polynucleotide with desirable binding characteristics if it actually exhibits linear binding.

The phrase "labeled population of nucleic acids" refers to mixture of nucleic acids that are detectably labeled, e.g., fluorescently labeled, such that the presence of the nucleic acids can be detected by assessing the presence of the label. A labeled population of nucleic acids is "made from" a chromosome composition, the chromosome composition is usually employed as template for making the population of nucleic acids.

A "non-cellular chromosome composition", as will be discussed in greater detail below, is a composition of chromosomes synthesized by mixing pre-determined amounts of individual chromosomes. These synthetic compositions can include selected concentrations and ratios of chromosomes that do not naturally occur in a cell, including any cell grown in tissue culture. Non-cellular chromosome compositions may contain more than an entire complement of chromosomes from a cell, and, as such, may include extra copies of one or more chromosomes from that cell. Non-cellular chromosome compositions may also contain less than the entire complement of chromosomes from a cell.

The term "array" encompasses the term "microarray" and refers to an ordered array presented for binding to nucleic acids and the like.

An "array," includes any two-dimensional or substantially two-dimensional (as well as a three-dimensional) arrangement of spatially addressable regions bearing nucleic acids, particularly oligonucleotides or synthetic mimetics thereof, and the like. Where the arrays are arrays of nucleic acids, the nucleic acids may be adsorbed, physisorbed, chemisorbed, or covalently attached to the arrays at any point or points along the nucleic acid chain.

Any given substrate may carry one, two, four or more arrays disposed on a front surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 cm² or even less than 10 cm², e.g., less than about 5cm², including less than about 1 cm², less than about 1 mm², e.g., 100 µ², or even smaller. For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 cm², or even less than 50 cm², 5 cm², 1 cm², 0.5 cm², or 0.1 cm². In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either nucleic acid precursor units (such as monomers) in the case *of in situ* fabrication, or the previously obtained nucleic acid. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Inter-feature areas need not be present particularly when the arrays are made by photolithographic methods as described in those patents.

An array is "addressable" when it has multiple regions of different moieties (e.g., different oligonucleotide sequences) such that a region (i.e., a "feature" or "spot" of the array) at a particular predetermined location (i.e., an "address") on the array will detect a particular sequence. Array features are typically, but need not be, separated by intervening spaces. In the case of an array in the context of the present application, the "population of labeled nucleic acids" will be referenced as a moiety in a mobile phase (typically fluid), to be detected by "surface-bound polynucleotides" which are bound to the substrate at the various regions. These phrases are synonymous with the terms "target" and "probe", or "probe" and "target", respectively, as they are used in other publications.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower" are used in a relative sense only.

The term "stringent assay conditions" as used herein refers to conditions that are compatible to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. Stringent assay conditions are the summation or combination (totality) of both hybridization and wash conditions.

A "stringent hybridization" and "stringent hybridization wash conditions" in the context of nucleic acid hybridization (e.g., as in array, Southern or Northern hybridizations) are sequence dependent, and are different under different experimental parameters. Stringent hybridization conditions that can be used to identify nucleic acids within the scope of the invention can include, e.g., hybridization in a buffer comprising 50% formamide, 5×SSC, and 1% SDS at 42°C, or hybridization in a buffer comprising 5×SSC and 1 % SDS at 65°C, both with a wash of 0.2×SSC and 0.1 % SDS at 65°C. Exemplary stringent hybridization conditions can also include a hybridization in a buffer of 40% formamide, 1 M NaCl, and 1% SDS at 37°C, and a wash in 1 ×SSC at 45°C. Alternatively, hybridization to filter-bound DNA in 0.5 M NaHPO₄, 7% sodium dodecyl sulfate (SDS), 1 mnM EDTA at 65°C, and washing in 0.1 ×SSC/0.1% SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions that set forth the conditions, which determine whether a nucleic acid is specifically hybridized to a probe. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2xSSC at a temperature of at least about 50°C or about 55°C to about 60°C for about 15 to about 20 minutes; or, the hybridization complex is washed twice with a solution with a salt concentration of about 2xSSC containing 0.1% SDS at oom temperature for 15 minutes and then washed twice by 0.1×SSC containing 0.1% SDS at 68°C for 15 minutes; or, equivalent conditions. Stringent conditions for washing can also be, e.g., 0.2×SSC/0.1% SDS at 42°C. In instances wherein the nucleic acid molecules re deoxyoligonucleotides ("oligos"), stringent conditions can include washing in 6×SSC/0.05% sodium pyrophosphate at 37°C (for 14-base oligos), 48°C (for 17-base oligos), 55°C (for 20-base oligos), and 60°C (for 23-base oligos). See Sambrook, Ausubel, or Tijssen (cited below) for detailed descriptions of equilvalent hybridization and wash conditions and for reagents and buffers, e.g., SSC buffers and equivalent reagents and conditions.

A specific example of stringent assay conditions is rotating hybridization at 65°C in a salt based hybridization buffer with a total monovalent cation concentration of 1.5M (e.g., as described in U.S. Patent Application No. 09/655,482 filed on September 5, 2000, the disclosure of which is herein incorporated by reference) followed by washes of 0.5X SSC and 0.1X SSC at room temperature.

Stringent hybridization conditions may also include a "prehybridization" of aqueous phase nucleic acids with complexity-reducing nucleic acids to suppress repetitive sequences. For example, certain stringent hybridization conditions include, prior to any hybridization to surface-bound polynucleotides, hybridization with Cot-1 DNA, or the like.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "pre-determined" refers to an element whose identity or composition is known prior to its use. For example, a "pre-determined chromosome composition" is a composition containing chromosomes of known identity. An element may be known by name, sequence, molecular weight, its function, or any other attribute or identifier.

The term "mixture", as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not especially distinct. In other words, a mixture is not addressable. To be specific, an array of surface bound polynucleotides, as is commonly known in the art and described below, is not a mixture of capture agents because the species of surface bound polynucleotides are spatially distinct and the array is addressable.

"Isolated" or "purified" generally refers to isolation of a substance (compound, polynucleotide, protein, polypeptide, polypeptide, chromosome, etc.) such that the substance comprises the majority percent of the sample in which it resides. Typically in a sample a substantially purified component comprises 50%, preferably 80%-85%, more preferably 90-95% of the sample. Techniques for purifying polynucleotides and polypeptides of interest are well known in the art and include, for example, ion-exchange chromatography, affinity chromatography, flow sorting, and sedimentation according to density.

The term "assessing" and "evaluating" are used interchangeably to refer to any form of measurement, and includes determining if an element is present or not. The terms "determining," "measuring," and "assessing," and "assaying" are used interchangeably and include both quantitative and qualitative determinations. Assessing may be relative or absolute. "Assessing the presence of" includes determining the amount of something present, as well as determining whether it is present or absent.

The term "using" has its conventional, and, as such, means employing, e.g. putting into service, a method or composition to attain an end. For example, if a program is used to create a file, a program is executed to make a file, the file usually being the output of the program. In another example, if a computer file is used, it is usually accessed, read, and the information stored in the file employed to attain an end. Similarly if a unique identifier, e.g., a barcode is used, the unique identifier is usually read to identify, for example, an object or file associated with the unique identifier.

Methods for evaluating surface-bound polynucleotides are provided. Specifically, the methods involve contacting an array of surface-bound polynucleotides with a population of labeled nucleic acids made from a non-naturally occurring composition of chromosomes, and evaluating binding of the labeled nucleic acids to a surface-bound polynucleotide. In most embodiments, binding is evaluated relative to binding of a second population of labeled nucleic acids made from a reference composition of chromosomes. The methods may be used to screen for surface bound polynucleotides that have desirable binding characteristics, e.g., suitability for use in array-based comparative genomic hybridization assays. Kits and computer programming for use in practicing the subject methods are also provided.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing the invention components that are described in the publications that might be used in connection with the presently described invention.

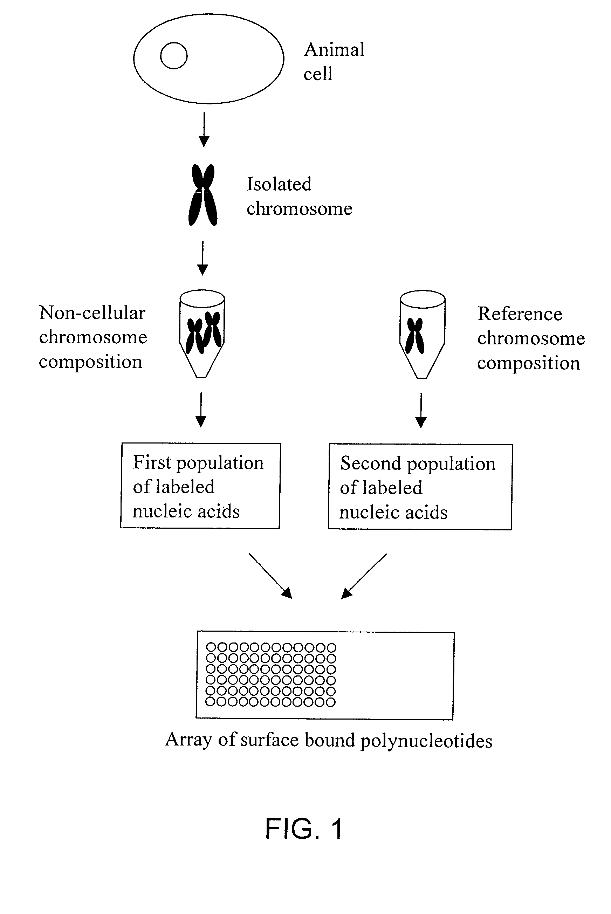

As summarized above, the present invention provides methods for evaluating surface-bound polynucleotides. With reference to Fig. 1, showing an exemplary embodiment of the invention, the methods usually involve obtaining a non-cellular chromosome composition and a reference chromosome composition, making a first and second population of labeled nucleic acids using those compositions, and contacting those populations of nucleic acids with an array of surface bound polynucleotides. As shown also shown in Fig. 1, the non-cellular chromosomal composition is a composition that usually contains at least one chromosome previously isolated from an animal cell, e.g., a human cell.

In further describing the present invention, chromosome compositions and arrays of surface-bound polynucleotides are described first, followed by a detailed description of the subject methods. Finally, representative kits and computer programming for use in practicing the subject methods will be discussed.

### Chromosome compositions

As mentioned above, the invention provides a variety of chromosome compositions that find use in the subject methods. In general, there are two types of chromosome compositions that find particular use in the subject methods: non-cellular chromosome compositions and reference chromosome compositions. Each of these chromosome compositions is described in greater detail below.

### Non-cellular chromosome compositions

A non-cellular chromosome composition is a composition containing a mixture of cellular chromosomes, at predetermined concentrations and/or ratios, that is not usually found in a cell, i.e., a mixture of chromosomes not naturally found in a cell, including cultured cells. For example, with respect to a particular cell, a non-cellular chromosome composition can have fewer chromosomes than a cell, or may contain chromosomes at relative levels not found in the cell. Accordingly, a non-cellular chromosome composition can also be thought of as a "non-naturally occurring" chromosome composition since it is never found in a cell, recombinant or otherwise.

In certain embodiments, a non-cellular chromosome composition may contain 1,2, 3, 4, 5, about 10, about 15 or about 20, up to 25 different chromosomes from a cell, as long as the composition contains fewer chromosomes than the cell. In other embodiments, a non-cellular chromosome composition may contain a mixture of different chromosomes of a cell, but at relative concentrations not found in that cell. For example, a non-cellular chromosome composition may contain at least one extra or at least one less copy (e.g., 1, 2, 3, 4, or 5 or more, usually up to about 10 extra or less copies) of a particular chromosome (or chromosomes) relative to the cell. In these embodiments, the non-cellular chromosome composition may or may not contain all of the different chromosomes of a cell, and, in certain embodiments, may contain only two different chromosomes. In addition some of these embodiments may include synthetic compositions that lack all copies of one or more chromosomes (e.g. a synthetic knockout) in the presence of one or more remaining chromosomes. Accordingly, a non-cellular chromosome composition may differ from the chromosome composition of a cell in that it contains chromosomes at a "ploidy", i.e., copy number, relative to other chromosomes in the composition that is not found in the cell.

Without wishing to limit the invention, the following examples are set forth to further describe non-cellular human chromosome compositions. These examples can be readily adapted to most non-cellular chromosome compositions for any animal since the number of chromosomes present can be simply adjusted to reflect the number of chromosomes present in that animal.

Human somatic cells contain 46 chromosomes, including 22 pairs of different autosomes and I pair of sex chromosomes (usually chromosomes X and Y, or two copies of chromosome X). Accordingly, the ratio between the different chromosomes of a human cell (i.e., the copy number of chromosome 1 relative to the copy number of chromosome 2) is usually 1:1. A cellular human chromosome composition (i.e., a composition found in the human cell and isolatable if the chromosomes of a human cell are separated from other components of the cell) contains all autosomal human chromosomes at a relative ratio of 1:1. The sex chromosomes, because they are not always present in two copies, can have a relative ratio of 1:1 (in XX cells) or 1:2 (in XY cells) as compared to autosomal chromosomes.

Accordingly, a non-cellular human chromosome composition may contain less than 22 different chromosomes. In certain embodiments, therefore, a non-cellular human chromosome composition may contain 1, 2, 3, 4, 5, about 8, about 10, about 15 or about 20, up to 21 different human chromosomes. A non-cellular human chromosome composition may also contain human chromosomes at ploidy levels different to those found in human cells (which, as discussed above, is usually 1:1). Accordingly, a non-cellular human chromosome composition may contain chromosomes, particularly autosomes (i.e., any one of human chromosomes 1-22), at a concentration of 0:1, 0.5:1, 2:1, 3:1, 4:1, 5:1, 6:1, 7:1, or more, usually up to 10:1, relative to another autosome in the reference chromosome composition. In some embodiments, therefore, the subject non-cellular chromosome compositions have one or more, e.g., two or three or more, autosomal chromosomes of a cell at relative amounts that are not found in the cell. In other embodiments the subject non-cellular chromosome compositions have one or two fewer, e.g. one or zero, autosomal chromosomes of a cell at relative amounts that are not found in the cell. Sex chromosomes may be or may not be present in such a composition.

Non-cellular chromosome compositions are usually pre-determined in that the chromosomes present in the compositions are usually defined prior to their use. In other words, non-cellular chromosome contain a fixed, non-variable and known composition, and contain known chromosomes at relative concentrations that are usually pre-determined prior to their use, or prior to their production. Relative concentrations of chromosomes in a non-cellular chromosome composition may be expressed as a ratio of whole numbers.

As illustrated in Fig. 1, non-cellular chromosome compositions are made from chromosomes isolated from a cell. In general, intact chromosomes from a cell are individually isolated and certain isolated chromosomes are selected and mixed together to form a non-cellular chromosome composition.

Methods for isolating chromosomes from a cell are very well known in the art (see, e.g., Gray et al., *High-speed chromosome sorting* Science (1987) 238 :323-9 and Hui et al., *Analysis of randomly amplified flow-sorted chromosomes using the* polymerase *chain reaction.* Genomics (1995) 26: 364-71) and need not be described here in any great detail. In general, intact chromosomes are isolated, stained, e.g., with Hoechst 33258 and chromomycin A3, and sorted using a flow cytometer with appropriate lasers and detection apparatus, on the basis of their staining. Accordingly, the chromosomes of a cell may be isolated from each other. Alternatively, individual chromosomes may be purchased from a supplier of chromosomes, e.g., FAST Systems Inc., (Gaithersburg MD).

In general, non-cellular chromosome composition may contain chromosomes from any cell of an organism with a genome that contains more than one chromosome, e.g., yeast, plants and animals, such as fish, birds, reptiles, amphibians and mammals. In certain embodiments, non-cellular mammalian chromosome compositions, i.e., those compositions containing chromosomes from mice, rabbits, primates, or humans, etc, can be made and used to evaluate and identify suitable surface bound polynucleotides.

Suitable cells that may be used as a source of mammalian chromosomes include: monkey kidney cells (COS cells), human embryonic kidney cells (HEK-293, Graham et al. J. Gen Virol. 36:59 (1977)); baby hamster kidney cells (BHK, ATCC CCL 10); chinese hamster ovary-cells (CHO, Urlaub and Chasin, Proc. Natl. Acad. Sci. (USA) 77:4216, (1980); mouse sertoli cells (TM4, Mather, Biol. Reprod. 23:243-251 (1980)); monkey kidney cells (CVI ATCC CCL 70); african green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (hep G2, HB 8065); mouse mammary tumor (MMT 060562, ATCC CCL 51); TRI cells (Mather et al., Annals N. Y. Acad. Sci 383:44-68 (1982)); NIH/3T3 cells (ATCC CRL-1658); and mouse L cells (ATCC CCL-1). Additional cells (e.g. human lymphocytes) and cell lines will become apparent to those of ordinary skill in the art, and a wide variety of cell lines are available from the American Type Culture Collection, 10801 University Boulevard, Manassas, Va. 20110-2209.

### Reference chromosome compositions

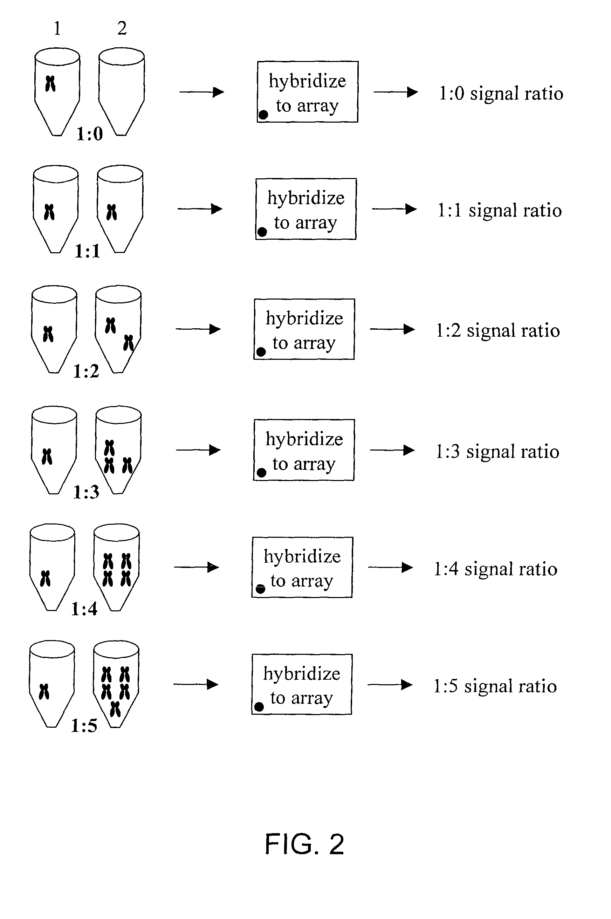

As mentioned briefly above and as shown in Fig. 2, a non-cellular chromosome composition is usually used in conjunction with a reference chromosome composition. A non-cellular chromosome composition and reference chromosome composition that are used together are termed a "chromosome composition pair" herein. As will be described in greater detail below, in use, the results obtained using a particular non-cellular chromosome composition are usually compared to the results obtained using a reference chromosome composition. Accordingly, within a chromosome composition pair, the reference chromosome composition usually contains one or more of the chromosomes of the non-cellular chromosome composition, but is different to the non-cellular chromosome composition.

A reference chromosome composition for a non-cellular chromosome composition generally contains more, or less chromosomes than the non-cellular chromosome composition. In many embodiments, therefore, the reference chromosome composition contains all of the different chromosomes present in the non-cellular chromosome composition, but at relative amounts that differ from the non-cellular chromosome composition. For example, if the non-cellular chromosome composition contains two different chromosomes at a certain non-natural ratio, e.g., 3:1, then the reference chromosome composition will typically contain the same two chromosomes at a different, known, ratio, e.g., 1:1. Accordingly, relative to a chromosome standard within each composition, the non-cellular and reference chromosome compositions within a chromosome composition pair typically have at least one chromosome (e.g., 1, 2, 3, 4, 5 or more, about 8 or more, about 10 or more, about 14 or more or about 20 or more chromosomes) in common, and that chromosome will be present in different amounts between the two compositions. In many embodiments therefore, a chromosome may be present in both compositions of a chromosome composition pair, but vary in relative amounts. A chromosome may be absent from, or present in an amount that is 0.5x (one half times, or half), 2x (two times, or twice), 3x, 4x, 5x, 6x, 7x, 8x or about 10x or more in, one composition as compared to the other. The relative amount of a chromosome in the two compositions of a chromosome composition pair is defined herein as a "chromosome ratio", which ratio is further discussed in the sections below.

In some embodiments the non-cellular chromosome composition and the reference chromosome composition have common genomic sequences present in equal concentrations. These sequences can consist of a portion of a chromosome, an entire chromosome, or multiple chromosomes. These enable the direct sample comparisons by providing signal intensity calibration across the two samples.

Since reference chromosome compositions may contain a composition of chromosomes that is not usually found in a cell, they may also be non-cellular chromosome compositions. Accordingly, such reference chromosome compositions may be made using the flow cytometry methods described above.

In other embodiments, certain reference chromosome compositions have a composition that is essentially identical to the composition of chromosomes found in a cell (in other words, they contain the same amounts of the same chromosomes). Accordingly, in certain embodiments, reference chromosome compositions may be made directly from a cell, by isolating a chromosomal extract from the cell. In these embodiments, there is no requirement that the individual chromosomes of the chromosomal extract be isolated, e.g., by cytometry or by any other means. If it is desirable, however, a reference composition having a composition that is identical to that of a particular cell may be "reconstituted" using isolated chromosomes.

Reference chromosome compositions typically contain about 2, 3, 4, 5, 6, 7, 8 about 10, about 12, about 15, about 20, about 25 or about 30 or more different chromosomes.

With specific reference to Fig. 2, a variety of chromosomal composition pairs, and a corresponding set of chromosome ratios suitable for use in the subject methods are shown. The tubes labeled "1" refer to a reference chromosome composition, and the tubes labeled "2" refer to a non-cellular chromosome composition,.

Accordingly, the invention provides a non-cellular chromosome composition containing at least two different chromosomes from a cell in relative amounts that are different to that found in that cell.

### Array platforms

Array platforms for performing the subject methods are generally well known in the art (e.g., see Pinkel et al., Nat. Genet. (1998) 20:207-211; Hodgson et al., Nat. Genet. (2001) 29:459-464; Wilhelm et al., Cancer Res. (2002) 62: 957-960) and, as such, need not be described herein in any great detail.

In general, arrays suitable for use in performing the subject methods contain a plurality (i.e., at least about 100, at least about 500, at least about 1000, at least about 2000, at least about 5000, at least about 10,000, at least about 20,000, usually up to about 100,000 or more) of addressable features that are linked to a usually planar solid support. Features on a subject array usually contain a polynucleotide that hybridizes with, i.e., binds to, genomic sequences from a cell. Accordingly, such "comparative genome hybridization arrays", for short "CGH arrays" typically have a plurality of different BACs, cDNAs, oligonucleotide primers, or inserts from phage or plasmids, etc., that are addressably arrayed. As such, CGH arrays usually contain surface bound polynucleotides that are about 10-200 bases in length, about 201-5000 bases in length, about 5001-50,000 bases in length, or about 50,001-200,000 bases in length, depending on the platform used.

In particular embodiments, CGH arrays containing surface-bound oligonucleotides, i.e., oligonucleotides of 10 to 100 nucleotides and up to 200 nucleotides in length, find particular use in the subject methods.

### Methods

The chromosome compositions described above are generally useful in methods of assessing a surface bound polynucleotide of interest. In general, the methods involve contacting a first population of labeled nucleic acids made from a non-cellular chromosome composition with an array of surface-bound polynucleotides, and evaluating a surface bound polynucleotide of interest for binding to the first population of labeled nucleic acids. In certain embodiments, evaluating is done relative to binding of the polynucleotide of interest to a population of nucleic acids made from a reference chromosome composition.

### Methods of assessing a surface-bound polynucleotide

In general, the subject methods of assessing a surface-bound polynucleotide involve labeling a non-cellular and a reference chromosomal composition to make two labeled populations of nucleic acids which may be distinguishably labeled, contacting the labeled populations of nucleic acids with at least one array of surface bound polynucleotides under specific hybridization conditions, and analyzing any data obtained from hybridization of the nucleic acids to the surface bound polynucleotides. Such methods are generally well known in the art (see, e.g., Pinkel et al., Nat. Genet. (1998) 20:207-211; Hodgson et al., Nat. Genet. (2001) 29:459-464; Wilhelm et al., Cancer Res. (2002) 62: 957-960)) and, as such, need not be described herein in any great detail.

In most embodiments, the chromosome compositions of a pair of chromosomal compositions (including any derivatives thereof, e.g., a chromosomal composition that contains fragmented or enzymatically amplified chromosomes, or amplified fragments of the same), are distinguishably labeled using methods that are well known in the art (e.g., primer, extension, random-priming, nick translation, etc.; see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.). The compositions are usually labeled using "distinguishable" labels in that the labels that can be independently detected and measured, even when the labels are mixed. In other words, the amounts of label present (e.g., the amount of fluorescence) for each of the labels are separately determinable, even when the labels are co-located (e.g., in the same tube or in the same duplex molecule or in the same feature of an array). Suitable distinguishable fluorescent label pairs useful in the subject methods include Cy-3 and Cy-5 (Amersham Inc., Piscataway, NJ), Quasar 570 and Quasar 670 (Biosearch Technology, Novato CA), Alexafluor555 and Alexafluor647 (Molecular Probes, Eugene, OR), BODIPY V-1002 and BODIPY V1005 (Molecular Probes, Eugene, OR), POPO-3 and TOTO-3 (Molecular Probes, Eugene, OR), fluorescein and Texas red (Dupont, Bostan MA) and POPRO3 TOPRO3 (Molecular Probes, Eugene, OR). Further suitable distinguishable detectable labels may be found in Kricka et al. (Ann Clin Biochem. 39:114-29, 2002).

The labeling reactions produce a first and second population of labeled nucleic acids that correspond to the non-cellular and reference chromosome compositions, respectively. After nucleic acid purification and any pre-hybridization steps to suppress repetitive sequences (e.g., hybridization with Cot-1 DNA), the populations of labeled nucleic acids are contacted to an array of surface bound polynucleotides, as discussed above, under conditions such that nucleic acid hybridization to the surface bound polynucleotides can occur, e.g., in a buffer containing 50% formamide, 5×SSC and 1% SDS at 42°C, or in a buffer containing 5×SSC and 1% SDS at 65°C, both with a wash of 0.2xSSC and 0.1% SDS at 65°C.

The labeled nucleic acids can be contacted to the surface bound polynucleotides serially, or, in other embodiments, simultaneously (i.e., the labeled nucleic acids are mixed prior to their contacting with the surface-bound polynucleotides). Depending on how the nucleic acid populations are labeled (e.g., if they are distinguishably or indistinguishably labeled), the populations may be contacted with the same array or different arrays. Where the populations are contacted with different arrays, the different arrays are substantially, if not completely, identical to each other in terms of target feature content and organization.

Standard hybridization techniques (using high stringency hybridization conditions) are used to probe a target nucleic acid array. Suitable methods are described in references describing CGH techniques (Kallioniemi et al., Science 258:818-821 (1992) and WO 93/18186). Several guides to general techniques are available, e.g., Tijssen, Hybridization with Nucleic Acid Probes, Parts I and II (Elsevier, Amsterdam 1993). For a descriptions of techniques suitable for in situ hybridizations see, Gall et al. Meth. Enzymol., 21:470-480 (1981) and Angerer et al. in Genetic Engineering: Principles and Methods Setlow and Hollaender, Eds. Vol 7, pgs 43-65 (plenum Press, New York 1985). See also United States Patent Nos: 6,335,167; 6,197,501; 5,830,645; and 5,665,549; the disclosures of which are herein incorporate by reference.

Generally, comparative genome hybridization methods comprise the following major steps: (1) immobilization of polynucleotides on a solid support; (2) pre-hybridization treatment to increase accessibility of support-bound polynucleotides and to reduce nonspecific binding; (3) hybridization of a mixture of labeled nucleic acids to the surface-bound nucleic acids, typically under high stringency conditions; (4) post-hybridization washes to remove nucleic acid fragments not bound to the solid support polynucleotides; and (5) detection of the hybridized labeled nucleic acids. The reagents used in each of these steps and their conditions for use vary depending on the particular application.

As indicated above, hybridization is carried out under suitable hybridization conditions, which may vary in stringency as desired. In certain embodiments, highly stringent hybridization conditions may be employed. The term "high stringent hybridization conditions" as used herein refers to conditions that are compatible to produce nucleic acid binding complexes on an array surface between complementary binding members, i.e., between the surface-bound polynucleotides and complementary labeled nucleic acids in a sample. Representative high stringency assay conditions that may be employed in these embodiments are provided above.

The above hybridization step may include agitation of the immobilized polynucleotides and the sample of labeled nucleic acids, where the agitation may be accomplished using any convenient protocol, e.g., shaking, rotating, spinning, and the like.

Following hybridization, the array-surface bound polynucleotides are typically washed to remove unbound labeled nucleic acids. Washing may be performed using any convenient washing protocol, where the washing conditions are typically stringent, as described above.

Following hybridization and washing, as described above, the hybridization of the labeled nucleic acids to the targets is then detected using standard techniques so that the surface of immobilized targets, e.g., the array, is read. Reading of the resultant hybridized array may be accomplished by illuminating the array and reading the location and intensity of resulting fluorescence at each feature of the array to detect any binding complexes on the surface of the array. For example, a scanner may be used for this purpose, which is similar to the AGILENT MICROARRAY SCANNER available from Agilent Technologies, Palo Alto, CA. Other suitable devices and methods are described in U.S. patent applications: Serial No. 09/846125 "Reading Multi-Featured Arrays" by Dorsel et al.; and United States Patent No. 6,406,849, which references are incorporated herein by reference. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). In the case of indirect labeling, subsequent treatment of the array with the appropriate reagents may be employed to enable reading of the array. Some methods of detection, such as surface plasmon resonance, do not require any labeling of nucleic acids, and are suitable for some embodiments.

Results from the reading or evaluating may be raw results (such as fluorescence intensity readings for each feature in one or more color channels) or may be processed results (such as those obtained by subtracting a background measurement, or by rejecting a reading for a feature which is below a predetermined threshold, normalizing the results, and/or forming conclusions based on the pattern read from the array (such as whether or not a particular target sequence may have been present in the sample, or whether or not a pattern indicates a particular condition of an organism from which the sample came).

In certain embodiments, the subject methods include a step of transmitting data or results from at least one of the detecting and deriving steps, also referred to herein as evaluating, as described above, to a remote location. By "remote location" is meant a location other than the location at which the array is present and hybridization occur. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart.

"Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

Accordingly, a pair of chromosome compositions is labeled to make two populations of labeled nucleic acids, the nucleic acids contacted with an array of surface-bound polynucleotides, and the level of labeled nucleic acids bound to each surface-bound polynucleotide is assessed.

In certain embodiments, a surface-bound polynucleotide is assessed by determining the level of binding of the population of labeled nucleic acids to that polynucleotide. The term "level of binding" means any assessment of binding (e.g. a quantitative or qualitative, relative or absolute assessment) usually done, as is known in the art, by detecting signal (i.e., pixel brightness) from the label associated with the labeled nucleic acids. Since the level of binding of labeled nucleic acid to a surface-bound polynucleotide is proportional to the level of bound label, the level of binding of labeled nucleic acid is usually determined by assessing the amount of label associated with the surface-bound polynucleotide.

In certain embodiments, a surface-bound polynucleotide may be assessed by evaluating its binding to two populations of nucleic acids that are distinguishably labeled. In these embodiments, for a single surface-bound polynucleotide of interest, the results obtained from hybridization with a first population of labeled nucleic acids may be compared to results obtained from hybridization with the second population of nucleic acids, usually after normalization of the data. The results may be expressed using any convenient means, e.g., as a number or numerical ratio, etc.

By "normalization" is meant that data corresponding to the two populations of nucleic acids are globally normalized to each other, and/or normalized to data obtained from controls (e.g., internal controls produce data that are predicted to equal in value in all of the data groups). Normalization generally involves multiplying each numerical value for one data group by a value that allows the direct comparison of those amounts to amounts in a second data group. Several normalization strategies have been described (Quackenbush et al, Nat Genet. 32 Suppl:496-501, 2002, Bilban et al Curr Issues Mol Biol. 4:57-64, 2002, Finkelstein et al, Plant Mol Bio).48(1-2):119-31, 2002, and Hegde et al, Biotechniques. 29:548-554, 2000). Specific examples of normalization suitable for use in the subject methods include linear normalization methods, non-linear normalization methods, e.g., using lowess local regression to paired data as a function of signal intensity, signal-dependent non-linear normalization, qspline normalization and spatial normalization, as described in Workman et al., (Genome Biol. 2002 3, 1-16). In certain embodiments, the numerical value associated with a feature signal is converted into a log number, either before or after normalization occurs. Data may be normalized to data obtained using the data obtained from a support-bound polynucleotide for a chromosome of known concentration in any of the chromosome compositions.

Accordingly, binding of a surface-bound polynucleotide to a labeled population of nucleic acids may be assessed. In most embodiments, the assessment provides a numerical assessment of binding, and that numeral may correspond to an absolute level of binding, a relative level of binding, or a qualitative (e.g., presence or absence) or a quantitative level of binding. Accordingly, a binding assessment may be expressed as a ratio, whole number, or any fraction thereof.

In other words, any binding may be expressed as the level of binding of a surface-bound polynucleotide to a labeled population of nucleic acids made from a non-cellular chromosome composition, divided by its level of binding to a labeled population of nucleic acids made from a reference chromosome composition (or vice versa).

### Methods of screening

The methods of assessing described above find use in methods of screening for surface-bound polynucleotides with binding characteristics that make them suitable for use in array-based comparative genome hybridization methods. Accordingly, the invention provides a method of screening in which binding of a candidate surface-bound polynucleotide is assessed using the methods described above, and surface-bound polynucleotides with desirable binding characteristics are identified.

In many embodiments, a surface-bound polynucleotide has desirable binding characteristics if data obtained using that polynucleotide corresponds to data expected for that polynucleotide. For example, candidate surface-bound polynucleotide binding may be assessed in a series of hybridization experiments using populations of labeled nucleic acids made from different non-cellular chromosomal compositions, as discussed above, and surface-bound polynucleotides may be screened on the basis of their level of binding to the labeled nucleic acids. Desirable surface-bound polynucleotides bind to the labeled nucleic acids to provide results consistent with the levels of particular chromosomes in the non-cellular and reference chromosome compositions, i.e., the "chromosome ratio", as discussed above.

This aspect of the invention may be described with reference to Fig. 2. Accordingly, with reference to Fig. 2, for a single surface-bound polynucleotide (illustrated by a filled circle on the arrays shown), a series of at least two assays (e.g., two, three, four, five at least about 7, at least about 10, usually up to about 20 or more assays) is performed using a population of labeled nucleic acids made from a reference chromosome composition (from the tubes marked "1") and a population of labeled nucleic acids made from a non-cellular chromosome composition (from the tubes marked "2"). Each assay uses different non-cellular chromosome compositions with pre-determined ratios of particular chromosomes (e.g., any chromosomes at any ratio, such as 1:0, 1:1, 1 :2, 2:3. 2:5, 3:5 etc.). The populations of labeled nucleic acids are hybridized to an array and results for each of the assays are obtained using the methods described above.

Surface-bound polynucleotides with desirable binding characteristics usually provide data, e.g., "signals" (i.e., assessments of binding) that correspond to pre-determined ratios of particular chromosomes in the chromosome compositions. For example, pairs of chromosome compositions having ratios of, e.g., 1:0, 1:1, 1:2, 1:3, 1:4, and 1:5, etc., would be expected to produce corresponding signal ratios of e.g., 1:0, 1:1, 1:2, 1:3, 1:4, and 1:5, etc., respectively. A surface-bound polynucleotide that provides data that is similar to, e.g., within about 5%, within about 10%, within about 15%, within about 20%, within about 30%, within about 40%, within about 50% etc., of expected ratios for that polynucleotide (based on the ratio of chromosomes corresponding to (i.e., that bind to) that polynucleotide in the chromosome compositions). Alternatively desirable binding characteristics of surface bound polynucleotides can be identified by the statistical significance (e.g. P value of Student's t test of less than 0.1, 0.01, 0.001, etc.,) of the separability of the distributions of their signals (e.g. ratios) in comparisons of two or more chromosome composition ratios in multiple repeat hybridizations (fig. 3). A surface-bound polynucleotide that provides signal ratios that are similar to the expected ratios is termed as surface-bound polynucleotide having "linear hybridization" properties.

Accordingly, by providing a method of assessing surface-bound polynucleotides, candidate surface-bound polynucleotides may be screened to identify surface-bound polynucleotides with desirable binding characteristics.

### Methods of producing an array

The methods described above provide surface-bound polynucleotides with desirable binding characteristics. Once such surface-bound polynucleotides with desirable binding characteristics, i.e., "validated" surface-bound polynucleotides, have been identified, they may be used to fabricate an array. Accordingly, the invention provides a method of producing an array. In general, the method involves identifying a surface-bound polynucleotide with desirable binding characteristic, and fabricating an array containing that polynucleotide.

A subject array may contain 1, 2, 3, more than about 5, more than about 10, more than about 20, more than about 50, more than about 100, more than about 200, more than about 500, more than about 1000, more than about 2000, more than about 5000 or more, usually up to about 10,000 or more, "validated" surface-bound polynucleotides.

Arrays can be fabricated using any means, including drop deposition from pulse jets or from fluid-filled tips, etc, or using photolithographic means. Either polynucleotide precursor units (such as nucleotide monomers), in the case of in situ fabrication, or previously synthesized polynucleotides (e.g., oligonucleotides, amplified cDNAs or isolated BAC, bacteriophage and plasmid clones, and the like) can be deposited. Such methods are described in detail in, for example U.S. patents 6,242,266, 6,232,072, 6,180,351, 6,171,797, 6,323,043, etc.

### Computer-related embodiments

The invention also provides a variety of computer-related embodiments. Specifically, the data analysis methods described in the previous section may be performed using a computer. Accordingly, the invention provides a computer-based system for analyzing data produced using the above methods in order to screen and identify surface-bound polynucleotide with desirable binding characteristics.

In most embodiments, the methods are coded onto a computer-readable medium in the form of "programming", where the term "computer readable medium" as used herein refers to any storage or transmission medium that participates in providing instructions and/or data to a computer for execution and/or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on computer readable medium, where "storing" means recording information such that it is accessible and retrievable at a later date by a computer.

With respect to computer readable media, "permanent memory" refers to memory that is permanent. Permanent memory is not erased by termination of the electrical supply to a computer or processor. Computer hard-drive ROM (i.e. ROM not used as virtual memory), CD-ROM, floppy disk and DVD are all examples of permanent memory. Random Access Memory (RAM) is an example of non-permanent memory. A file in permanent memory may be editable and re-writable.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

### Kits

Also provided by the subject invention are kits for practicing the subject methods, as described above. The subject kits at least include a non-cellular chromosome composition comprising at least one isolated chromosome, and a reference chromosome composition comprising a reference chromosome. Other optional components of the kit include: nucleic acid labeling agents, such as for primer extension or nick translation and fluorescent labels conjugated to nucleotides. In some embodiments, arrays may be included in the kits. In alternative embodiments, the kit may also contain computer-readable media for performing the subject methods, as discussed above. The various components of the kit may be present in separate containers or certain compatible components may be precombined into a single container, as desired.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

In addition to the subject database, programming and instructions, the kits may also include one or more control analyte mixtures, e.g., two or more control analytes for use in testing the kit.

### Utility

The subject methods find most application in identifying surface-bound polynucleotides, e.g., BACs, cDNAs, oligonucleotides, etc., suitable for use in CGH assays, e.g, any application in which one wishes to compare the copy number of nucleic acid sequences found in two or more genomic samples. Once identified, surface-bound polynucleotides suitable for use in CGH assays may be used to make a CGH array. Such a CGH array may be used in CGH assays to obtain high quality, reliable, data that is free from the artifacts (e.g. compression of observed ratios due to crosshybridization of surface-bound polynucleotides with non-target sequences) commonly obtained using CGH arrays containing surface-bound polynucleotides identified using other methods. Accordingly, the subject methods find use in making CGH arrays.

One type of representative application in which the subject CGH arrays find use is the quantitative comparison of copy number of one nucleic acid sequence in a first collection of nucleic acid molecules relative to the copy number of the same sequence in a second collection.

As such, the present invention may be used in methods of comparing abnormal nucleic acid copy number and mapping of chromosomal abnormalities associated with disease. In many embodiments, the subject methods are employed in applications that use polynucleotides immobilized on a solid support, to which differentially labeled nucleic acids produced as described above are hybridized. Analysis of processed results of the described hybridization experiments provides information about the relative copy number of nucleic acid domains, e.g. genes, in genomes.

Such applications compare the copy numbers of sequences capable of binding to the target elements. Variations in copy number detectable by the methods of the invention may arise in different ways. For example, copy number may be altered as a result of amplification or deletion of a chromosomal region, e.g. as commonly occurs in cancer.

Representative applications in which the subject methods find use are further described in U.S. Patent Nos. 6,335,167; 6,197,501; 5,830,645; and 5,665,549; the disclosures of which are herein incorporated by reference.

The following examples are offered by way of illustration and not by way of limitation.

### EXPERIMENTAL

### MATERIALS AND METHODS:

Genomic DNA specific for each human chromosome was obtained from a commercial supplier. Individual chromosome samples were quantified by standard fluorescence measurements for DNA, i.e., 260/280 nm absorbance after amplification with the phi29 polymerase and restriction digestion. All digests were done with AluI and RsaI according to the manufacturer's instructions (Promega) then verified by agarose gel analysis. Individual reference and experimental samples were then filtered using the Qiaquick PCR Cleanup Kit (Qiagen). Non-cellular compositions containing the equivalent of 4 copies of chromosome 17 and 2 copies of all other chromosomes were prepared. A reference genome mixture was prepared by mixing aliquots of each chromosome equivalent to their 2 copy representation in the human genome.

**Sample labeling.** Labeling reactions were performed with purified restricted DNA and a Bioprime labeling kit (Invitrogen) according to the manufacturer's directions in a 50 µl volume with a modified dNTP pool; 120 µM each of dATP, dGTP, dTTP, 60 µM dTTP, and 60 µm of either Cy5-dUTP for the experimental sample or Cy3-dUTP for the 46,XX female reference (Perkin-Elmer, Boston, MA). Labeled targets were subsequently filtered using a Centricon YM-30 filter (Millipore, Bedford, MA). Experimental and reference targets for each hybridization were pooled, mixed with 50 µg of human Cot-1 DNA (Invitrogen), 100 µg of yeast tRNA (Invitrogen) and 1X hybridization control targets (SP310, Operon). The target mixture was purified then concentrated with a Centricon YM-30 column, and resuspended to a final volume of 250 µl, then mixed with an equal volume of Agilent 2X *in situ* Hybridization Buffer.

Prior to hybridization to the array, the 500 µl hybridization mixtures were denatured at 100°C for 1.5 minutes and incubated at 37°C for 30 minutes. In order to remove any precipitate, the mixture was centrifuged at ≥ 14,000 g for 5 minutes and transferred to a new tube leaving a small residual volume (≤ 5 µl). The sample was applied to the array using an Agilent microarray hybridization chamber and hybridization was carried out for 14-18 hrs at 65°C in a Robbins Scientific rotating oven at 4 rpm. The arrays were then disassembled in 0.5X SSC/0.005% Triton X102 (wash 1) at 65°C then washed for 10 minutes at RT in wash 1, followed by 5 minutes at RT in 0.1 X SSC/0.005% Triton X102 (wash 2). Slides were dried and scanned using an Agilent 2565AA DNA microarray scanner.

### RESULTS:

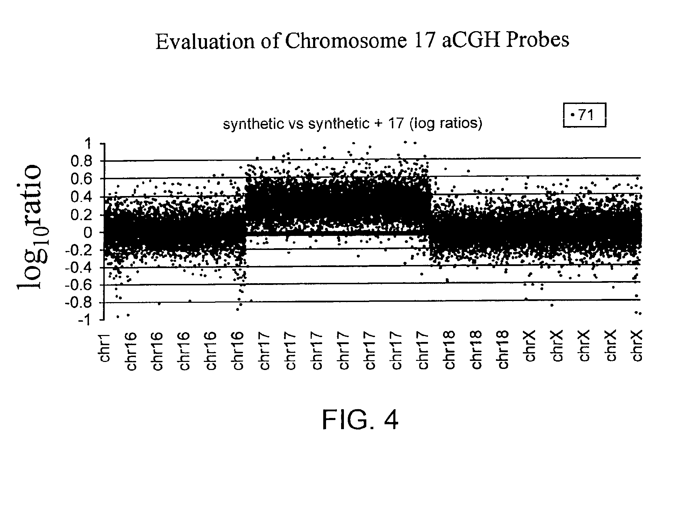

Test samples containing the equivalent of 4 copies of chromosome 17 and normal ( i.e. 2) copies of chromosome 16, 18 and X, and a reference sample with 2 copies of each of these chromosomes were hybridized to an oligonucleotide array with a high density of probes specific for chromosomes 16, 17, 18 and X. The test sample composition recreates a tetraploid (4 copies) chromosome 17 test sample that does not occur naturally. The ratios of test/reference signals for chromosomes 16, 18 and X probes were centered on a log value of 0 (i.e. 1). In contrast the ratios for chromosome 17 probes were centered on a log value of 0.3 (i.e. 2). Thus, hybridization of this composition with a reference sample identified chromosome 17 specific probes that have desired binding characteristics (e.g. ratios of log = 0). Fig. 4 shows the results of these experiments and allows an assessment of the surface bound polynucleotides.

The above results and discussion demonstrate a new method for screening for surface bound polynucleotides with desirable binding characteristics. Such methods are superior to currently used methods because they provide a way of testing CGH probes using chromosome mixtures of known composition without the need for growing particular cell lines with altered chromosome numbers. Further, once made, the chromosome compositions are fixed and may be re-used for several assays, in contrast to cell lines that have variable ploidy levels. Finally, in contrast to known methods, the subject methods may be used to test surface bound polynucleotides that correspond to, or bind to, any chromosome of a cell since non-cellular chromosome compositions may be made with any amount of any of the chromosomes, particularly autosomal chromosomes, of a cell. As such, the subject methods represent a significant contribution to the art.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The disclosures in United States patent application No. 10/828,907, from which this application claims priority, and in the abstract accompanying this application are incorporated herein by reference.

## Claims

1. A method of assessing a surface-bound polynucleotide, comprising:
contacting a first labeled population of nucleic acids made from a non-cellular chromosome composition with an array of surface-bound polynucleotides; and
evaluating binding of a surface-bound polynucleotide to said first labeled population of nucleic acids relative to binding of a second labeled population of nucleic acids made from a reference chromosome composition.

2. A method as claimed in claim 1, wherein said non-cellular chromosome composition contains at least one, but less than all of the, chromosomes from a mammalian cell.

3. A method as claimed in claim 2, wherein said at least one chromosome is present at a relative level that does not naturally occur in said mammalian cell.

4. A method as claimed in claim 1, wherein said non-cellular chromosome composition contains all of the chromosomes of a mammalian cell, with one or more chromosomes present in an amount that does not naturally occur in said mammalian cell.

5. A method as claimed in any preceding claim, wherein said surface-bound polynucleotide is an oligonucleotide.

6. A method of assaying a candidate surface-bound polynucleotide for suitability for use in array-based comparative genome hybridization assays, comprising:
assessing binding of said candidate surface-bound polynucleotide on an array according to the method of any of claims 1 to 5.

7. A method as claimed in claim 6, wherein a surface-bound polynucleotide suitable for use in array-based comparative genome hybridization assays is a surface-bound polynucleotide that binds to said first and second labeled nucleic acid populations at a relative level that corresponds to the relative level of a chromosome in said chromosome compositions.

8. A method of producing an array, comprising,
identifying a surface-bound polynucleotide suitable for use in array-based comparative genome hybridization assays according to the method of any of claims 1 to 7; and
fabricating an array comprising said surface-bound polynucleotide.

9. An array of surface-bound polynucleotides, wherein at least one of said surface-bound polynucleotide has been identified using the method of claim 8.

10. A kit comprising:
a non-cellular chromosome composition comprising at least one chromosome isolated from an animal cell; and,
a reference chromosome composition comprising a reference chromosome;
wherein said chromosome isolated from an animal cell and said reference chromosome are the same chromosome and are present in said compositions at pre-determined relative amounts.
